# EUROPÄISCHE PATENTSCHRIFT

(11) **EP 1 399 275 B1**
(45) Veröffentlichungstag und Bekanntmachung des Hinweises auf die Patenterteilung: **21.04.2010**
(21) Anmeldenummer: 02745151.7
(22) Anmeldetag: 26.06.2002
(51) Int. Cl.: B09B 1/00, B09B 3/00, C05F 17/02

(54) **VERFAHREN UND VORRICHTUNG ZUR BESCHLEUNIGUNG DES ABBAUS BIOGENER ORGANIK IN ABFALLDEPONIEN**
METHOD AND DEVICE FOR ACCELERATING THE DECOMPOSITION OF BIOGENIC ORGANIC MATTER IN REFUSE DISPOSAL SITES
PROCEDE ET DISPOSITIF POUR ACCELERER LA DECOMPOSITION DE LA MATIERE ORGANIQUE BIOGENE DANS DES DECHARGES A ORDURES

(30) Priorität: 29.06.2001 DE 10131026
(43) Veröffentlichungstag der Anmeldung: 24.03.2004
(73) Patentinhaber: CDM Consult GmbH, 44793 Bochum (DE); Stadtreinigung Hamburg, 20537 Hamburg (DE)
(72) Erfinder: KANITZ, Jürgen, 44805 Bochum (DE); FORSTING, Jürgen, 48149 Münster (DE)
(74) Vertreter: Vomberg, Friedhelm
(86) Internationale Anmeldenummer: PCT/DE2002/002331
(87) Internationale Veröffentlichungsnummer: WO 2003/004182

(56) Entgegenhaltungen:
- EP-A1- 0 505 218
- AT-B- 403 480
- DE-A- 10 005 243
- US-A- 4 837 153
- US-A- 5 632 798
- US-A- 5 642 630
- US-A- 6 024 513
- KNAUF S ET AL: "KOMPOSTMIETEN - GEGENUEBERSTELLUNG VERSCHIEDENER BELUEFTUNGSVARIANTEN" KORRESPONDENZ ABWASSER, ABWASSERTECHNISCHE VEREINIGUNG, ST. AUGUSTIN, DE, Bd. 43, Nr. 3, 1. März 1996 (1996-03-01), Seiten 428-432,434-435, XP000692899 ISSN: 0341-1540

## Beschreibung

Die Erfindung betrifft ein Verfahren zur Beschleunigung des Abbaus biogener Organik in Abfalldeponien, in denen die Abfälle in einem auf einem Deponiegrund aufgeschichteten Haufwerk gelagert sind, in das mindestens eine Saugleitung eingezogen ist, über die in dem Haufwerk entstehende Deponiegase abgesaugt werden.

Die Erfindung betrifft ferner eine Vorrichtung zur Durchführung des vorgenannten Verfahrens, mit mindestens einer in das Haufwerk eingezogenen, sich bis zum Deponiegrundbereich erstreckenden Saugleitung, mindestens einem Messfühler zur Analyse und Messung der Konzentration der im Haufwerk entstehenden Gase und einer Steuervorrichtung zur Einstellung der Saugleistung, nämlich der Durchflussmenge der abgesaugten Gase in der oder den Saugleitungen in Abhängigkeit der über den oder die Messfühler festgestellten Gase und deren Konzentrationen.

Unter Abfällen werden solche Gemische verstanden, die aus Hausmüll oder hausmüllähnlichen Gewerbeabfällen bestehen und die neben nicht zersetzbaren anorganischen Bestandteilen organische Bestandteile aufweisen, die sich bei Anwesenheit von Sauerstoff und einer hinreichenden Feuchtigkeit aerob und bei Abwesenheit von Sauerstoff anaerob zersetzen.

Erst in jüngster Zeit wurden durch die Abfalltrennung die Voraussetzungen geschaffen, gezielt im Wege einer (aeroben) Kompostierung oder in anaeroben Prozessen (Faultürmen oder ähnlichem) eine Abfallverwertung und/oder - beseitigung vorzunehmen, um bei ständig knapper werdendem Deponieraum eine möglichst umweltverträgliche Abfallentsorgung zu gewährleisten. Vollständig aerob abgebauter organischer Abfall kann als Kompost verwendet werden. In einer anaeroben Abfallbehandlung kann das gewonnene Methan als verwertbare Energie genutzt werden. Entsprechendes gilt auch, soweit der Abfall hinreichend zu einem Stabilat getrocknet ist und unter Ausnutzung des Brennwertes verbrannt wird.

Insbesondere in früheren Jahren sind jedoch anfallende Abfälle zu Müllhalden aufgeschichtet oder in vorhandene oder geschaffene Gruben abgekippt worden. Nach Erschöpfung der Kapazität und dem Schließen dieser Deponien sind die Haufwerke mehr oder weniger abgedeckt und in Einzelfällen begrünt worden. Die sich zwangsläufig in dem aufgeschichteten Haufwerk einstellende anaerobe Zersetzung der organischen Bestandteile führt zu einer Methangasbildung, die insbesondere in städtischen oder stadtnahen Bereichen intolerable Konzentrationen aufwies.

In solchen abgeschlossenen Deponien sind daher Saugleitungen eingezogen worden, über die entstehendes Deponiegas abgesaugt wurde. Die anfänglich hohen Methangaskonzentrationen von bis zu 60 Vol% lassen eine wirtschaftliche Absaugung und Verbrennung des Methans in Blockheizkraftwerken zu. Mit der Zeit verlangsamt sich jedoch der Abbauprozess, so dass der prozentuale Methangasgehalt im abgesaugten Deponiegas deutlich geringer wird, so dass schließlich Konzentrationen erreicht werden, die eine wirtschaftliche Verbrennung einerseits ausschließt, andererseits noch Werte von beispielsweise bis zu 25Vol% erreichen, die Gefährdungen durch Explosionen in geschlossenen Räumen bewirken können. Über Gasmigration können so größere Entfernungen (bis zu 300 m sind dem Verfasser bekannt) überbrückt werden, bevor das Gas z.B. in Kellerräumen oder Abwasserschächten austritt und hier gefährliche, explosive Gasgemische bildet.

Um diese Gefährdung zu minimieren, ist nach dem Stand der Technik die Deponiegasabsaugung noch mehrere Jahre vorzunehmen. Hierzu werden über vorhandene Saugleitungen, Brunnen oder ähnliches über die gesamte Haufewerkhöhe Deponiegasabsaugungen vorgenommen, wobei die Saugleistung der Menge des entstehenden Deponiegases entsprechend angepasst reguliert wird.

Sinken die CH4-Konzentrationen dabei unter ca. 25-27 Vol%, ist die Brennbarkeit des Gases nicht mehr gegeben. Entweder muss mittels Gaszumischung die CH4-Konzentration auf über 30 Vol% angehoben werden um das Gasgemisch zu verbrennen oder das Gasgemisch wird ohne Gaszumischung z.B. in einer Katalysatorstufe zu CO₂ oxidiert.

Die US-A 5632798 beschreibt ein Verfahren zur aeroben Kompostierung von organischem Müll, bei dem der organische Müll in einen Sack gegeben und belüftet wird.

Die US-A 4837153 beschreibt Lanzen zum Einlassen oder Absaugen von Luft oder Flüssigkeiten.

Die US-A 6024513 offenbart ein Verfahren zur Kompostierung von Hausmüll in einer Aufschüttung, die gleichzeitig befeuchtet und über mehrere Lanzen mit Gas durchströmt wird.

S. Knauf et al, in Korrespondenz Abwasser vom 1. März 1996, Bd. 43, Nr. 3, Seite 428-435, "Kompostmieten - Gegenüberstellung verschiedener Belüftungsvarianten" behandelt die Unterschiede zwischen einer Saug- und einer Druck-Belüftung in Rotteboxen.

Nach dem in der AT 403 480 B beschriebenen Verfahren zum mikrobiologischen Abbau und/oder der Stabilisierung von zumindest zeitweise anaerob fermentierten Gemischen in Deponien soll die durch eine Abtragung geschaffene Oberfläche vor und/oder nach dem Verlegen von Fluidleitungen in wesentlichen fluiddicht abgedichtet werden, wonach über eingezogene Leitungen ein sauerstoffhaltiges Gas in zur Abtragung vorgesehene Bereiche injiziert wird.

In der US-A 5642630 wird ein Verfahren zur Förderung und Trennung von Methan und Kohlendioxid aus Deponiekörpern beschrieben. Hierbei wird der Saugdruck so eingestellt, dass eine Migration der atmosphärischen Luft in die Oberflächenschichten vermieden wird.

Die EP 0 505 218 A zeigt und beschreibt eine mit Öffnungen ausgestattete Lanze zur Methangasabsaugung.

Auf Grund der üblicherweise ausgestalteten Gasbrunnen mit einer bis nahezu zur Oberfläche reichenden Verfilterung, führt eine stärkere Saugung nur zu einer oberflächennahen Belüftung und einer ggf. Umsetzung der biogenen Organik. In tieferen Schichten, dem größten Anteil des Deponats erfolgt keine Änderung der Umsetzungsbedingungen. Durch den biologischen Abbau gehen andererseits infolge eines Hydrolyseprozesses organische Verbindungen in eine wassergelöste Form über und werden über das Sicherwasser abtransportiert. Sie führen somit zu einer möglichen Grundwasserbelastung (gemessen als CSB- und NH4-Gehalt im Sickerwasser). Nach derzeitigem Kenntnisstand dauert die Gefahr der Gasemissionen sowie die der Sickerwasseremissionen zwischen 50 und 100 Jahren.

Es ist daher Aufgabe der vorliegenden Erfindung, ein Verfahren und eine Vorrichtung der eingangs genannten Art anzugeben, womit unter Minimierung des ansonsten zu treibenden Aufwandes ein massiv beschleunigter Abbau der biogenen Organik in Abfalldeponien erreicht werden kann. Die Laufzeit bis zum quasi vollständigen Abbau der bioabbaubaren Organik sollte deutlich reduziert werden, im Regelfall unter 10 Jahre.

Diese Aufgabe wird durch das Verfahren nach Anspruch 1 sowie durch die Vorrichtung nach Anspruch 5 gelöst; erfindungsgemäße Weiterentwicklungen sind in den Unteransprüchen 2 bis 4 bzw. 6 bis 11 beschrieben.

Der Kerngedanke der vorliegenden Erfindung beruht darauf, dass in dem Haufwerk aerobe Bedingungen eingestellt werden, unter denen die biologischen Abbauprozesse wesentlich schneller ablaufen als unter anaeroben Bedingungen. Durch die Maßnahme, dass im Bereich des Deponiegrundes die abgesaugte Gasmenge größer ist als die in diesem Bereich durch Zersetzung entstehende Gasmenge, werden in Abhängigkeit von der Saugleistung nach einiger Zeit zwangsweise aerobe Bedingungen im aufgeschichteten Haufwerk hergestellt. Über den Deponiemantel wird über die Außenluft Sauerstoff angesogen. Anders als bei den nach dem Stand der Technik bekannten Absaugeinrichtungen findet die Absaugung im unteren Deponiebereich, vorzugsweise im unteren Drittel statt, d.h. vorzugsweise in einer Zone, die sich im allgemeinen bis zu einer Höhe von maximal 6 m über dem Deponiegrund in noch gaserfüllten Bereich befindet. Die Absaugung soll in jedem Fall basisnah erfolgen und - soweit möglich - nicht nach oben über den genannten Bereich ausgedehnt werden. Die Höhe der Absaugzone hängt jedoch auch von der Höhe des aufgeschichteten Haufwerkes ab. Die Deponie wird übersaugt, wobei wenige Saugpegel im Zentralbereich der Deponie so angeordnet sind, dass über die Saugung auf das Deponat ein annähernd konstanter Unterdruck angelegt wird. Die Deponie wird so übersaugt, dass die Strömungsgeschwindigkeit im Deponat von den Saugpegeln zu den Außenbereichen, in denen Außenluft einströmt stark abnimmt. Aufgrund der geringen Strömungsgeschwindigkeit in großen Flächen kommt es nicht zu lokalen Überhitzungen, der Bildung von Schwundrissen oder sonstigen Kanälen, über die bevorzugt Luft einströmt.

Dies ist vorwiegend der Fall, wenn bei den nach dem Stand der Technik bekannten Verfahren über Lanzen oder Gasbrunnen Luft in das Deponat gedrückt wird. Am Eintragsort besteht, in einem vergleichsweise kleinen Deponatvolumen durch die große Strömung ein hohes Luftsauerstoffdargebot. Es kommt zu einer intensiven, lokalen biologischen und chemischen Umsetzung der biogenen Organik. Es kommt somit im Bereich der Einträge zu lokalen Überhitzungen, teilweise bis hin zu Glutnestern. Aufgrund der Temperaturerhöhung nimmt das Gas sehr viel mehr Feuchtigkeit auf, als es über die eingetragene kältere Luft eingebracht werden kann. Das Deponat trocknet teilweise aus, die biologische Umsetzung geht zurück. Durch das Austrocknen kommt es teilweise zu Schwundrissen und somit trotz intensiver Belüftung zu unkontrolliertem Abstrom der eingetragenen Luft.

Erfindungsgemäß wird durch die gleichmäßige Belüftung des gesamten Haufwerkes ein Sauerstoffüberschuss geschaffen, durch den eine aerobe Umsetzung bewirkt wird, deren gasförmiges Endprodukt CO₂ und H₂O ist. Es ist auch wesentlich, die Gasabsaugung möglichst tief am Deponiegrund, vorzugsweise im unteren Drittel vorzunehmen, da ansonsten die Gefahr besteht, dass die gesamte, angesaugte Luftmenge in den obersten Schichten des Deponie-Haufwerkes über dort vorhandene Saugschlitze unter Passieren nur geringer Deponatvolumina abgezogen wird, wodurch zwar im oberen Bereich eine Aerobisierung und Zersetzung ermöglicht wird, sich jedoch in den unteren Zonen des Haufwerkes bis hin zum Deponiefuß an der aneroben Zersetzung nichts ändert. Bei der erfindungsgemäßen Aerobisierung wird einerseits durch Einstellung der aeroben Bedingungen die Methanbildung reduziert und andererseits die aerobe Umsetzung zu CO₂ stark erhöht. Eine hohe CO₂-Konzentration bei niedrigen Methan-, aber auch niedrigen Sauerstoffkonzentrationen ist ein meßbares Indiz für eine gute Aerobisierung im Anfangsstadium.

Das erfindungsgemäße Verfahren lässt sich dann auch mit bisher praktizierten Maßnahmen zur Methangasgewinnung derart kombinieren, wenn zunächst bei vorhandenen Saugleitungen oder Brunnen, durch geeignete technische Maßnahmen der Filter- und Absaugbereich in den untersten Bereich, vorzugsweise das untere Drittel umgerüstet werden. Deponiegas wird danach mit entsprechend hohem Methangehalt so lange abgepumpt, bis der Methangehalt im Gas auf einen Wert absinkt, ab dem eine wirtschaftliche Nutzung des Methans, z.B. durch Verbrennung in einem Blockheizkraftwerk, nicht mehr gegeben ist.

Soweit vorgesehen ist, dass in unterschiedlichen Höhen Messfühler (Filterkerzen) im Brunnen (Multilevelbrunnen), aber außerhalb des eingezogenen Rohres verbleiben sollen, sind diese Filterkerzen in einem Filterkiesbett angeordnet. Zwischen diesen Filterkiesbetten bzw. den Filterkerzen sind Tonsperren vorgesehen, so dass gewährleistet ist, dass in unterschiedlichen Deponiehöhen jeweils über diese Messfühler die Deponiegasentwicklung durch Gasprobenahmen kontrolliert werden kann.

Die Deponiegasentwicklung und deren Zusammensetzung zeigt dann an, ob die gewünschte Absaugleistung groß genug gewählt worden ist, um das Deponiehaufwerk über das gesamte Volumen hinreichend zu belüften.

Nach einer weiteren Ausgestaltung der Erfindung wird die Gasabsaugung ausschließlich im Bereich des Deponiegrundes, vorzugsweise im unteren Drittel des Haufwerks und/oder in einer sich vorzugsweise bis maximal 6 m über den Deponiegrund erstreckenden Zone im gaserfüllten Bereich (ungesättigter Bereich) durchgeführt. Zumindest im wesentlichen soll oberhalb der vordefinierten Zone keine Gasabsaugung stattfinden, es sei denn, es handelt sich um kleinere, über etwaige Filterkerzen entnommene Gasproben, mit denen die Gaszusammensetzung in Abhängigkeit der Haufwerkhöhe untersucht wird, um hiernach die Absaugleistung im unteren Deponiebereich zu erhöhen oder zu erniedrigen.

Vorzugsweise wird die Gasabsaugung an mehreren, im Deponiegrundbereich angeordneten Orten, die weiterhin vorzugsweise äquidistant zueinander liegen, durchgeführt. Hiermit wird dem Umstand Rechnung getragen, dass sich Deponien zumeist großflächig erstrecken, so dass entsprechend viele Saugleitungen bzw. Brunnen zweckmäßig sind.

Wie bereits vorstehend am Beispiel sogenannter Multilevelbrunnen angedeutet, wird die Gaszusammensetzung in unterschiedlichen Höhen im Haufwerk an verschiedenen Orten gemessen, die Messung ausgewertet und die Saugleistung in Abhängigkeit hiervon gesteuert, so dass sich an jedem Ort im Haufwerk ein für eine aerobe Zersetzung notwendiger Sauerstoffgehalt einstellt. Verwendet man mehrere Brunnen oder sonstige Saugleitungen, kann individuell in jeder Saugleitung bzw. in jedem Brunnen die Durchflussmengen des abgesaugten Deponiegases variiert werden, um Bereiche mit noch anaerober Zersetzung stärker zu belüften und um die aerobe Zersetzung zu optimieren.

Das beschriebene Verfahren wird mittels der Vorrichtung nach Anspruch 5 durchgeführt, die erfindungsgemäß dadurch gekennzeichnet ist, dass die Absaugöffnung der Saugleitung(en) im Bereich des Deponiegrundes angeordnet ist/sind, nämlich in einem Bereich, der sich im unteren Drittel des Haufwerks bzw. in einem Bereich, der sich vom Deponiegrund erstreckt. Vorzugsweise sind in das Haufwerk mehrere, insbesondere zueinander äquidistant angeordnete Saugleitungen eingezogen. In einer weiteren Ausgestaltung der Erfindung sind mehrere Saugleitungen so installiert, dass deren Lage, Abstand und Saugleistung derart gewählt ist, dass sich die Absaugwirkbereiche zumindest berühren, vorzugsweise geringfügig überlappen. Insbesondere ist die Saugleistung in jeder Saugleitung individuell regelbar, vorzugsweise in Abhängigkeit der in deren betreffendem Wirkbereich gemessenen Gaskonzentration steuerbar. Die Saugleitungen besitzen in ihrem oberen Bereich (in dem kein Deponiegas abgesaugt werden soll) einen gasdichten Mantel sowie eine außenliegende Tonsperre, um einen Gaskurzschluss parallel zum ungeschlitzten Saugrohr zu verhindern.

Die vorliegende Erfindung wird anhand eines Ausführungsbeispiels erläutert. Es zeigen
- Fig. 1: einen Querschnitt durch eine Grubendeponie und
- Fig. 2: einen Teilquerschnitt durch einen Multilevel-Brunnen

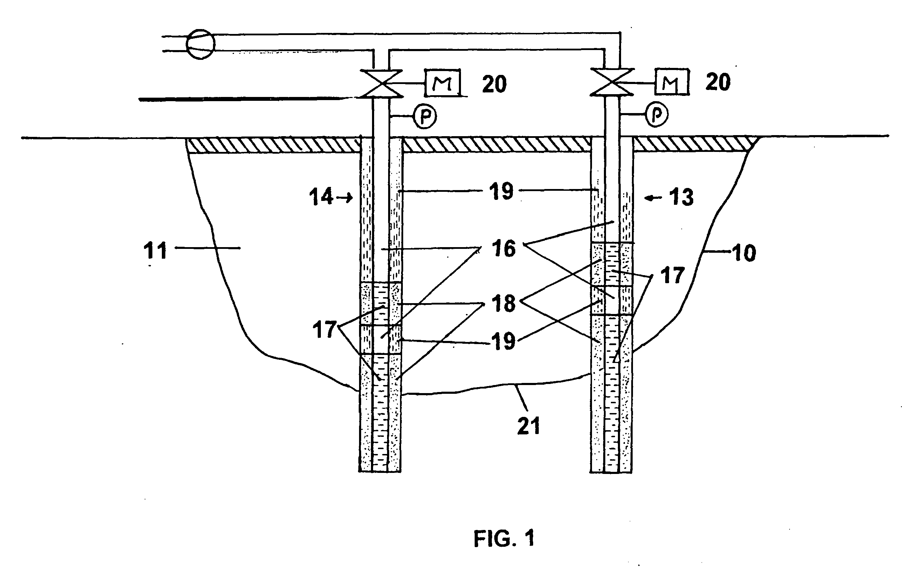

Gemäß Fig. 1 sind in einer Grube (10) Abfälle zu einem Haufwerk (11) (Deponat) aufgeschichtet, das nach oben durch eine natürliche Abdeckung (12), z.B. in Form einer Deponiebegrünung abgeschlossen wird. Im dargestellten Ausführungsbeispiel sind zwei Bohrungen (13) und (14) vorgesehen, in die jeweils Rohre (16) mit einem geschlossenen Mantel eingezogen sind. Im vorliegenden Fall sind ferner zwei Filterrohre (17) vorgesehen, um die herum ein gasdurchlässiges Kiesbett (18) angeordnet ist. Darüber bzw. dazwischen liegende Tonsperren (19) sollen verhindern, dass Gas unter Umgehung des Deponats am Vollrohr vorbei bzw. durch die Filterkiessschicht direkt in den Absaugbrunnen gesaugt wird und somit die Effektivität der Saugbrunnen (Saugregionen) vermindert wird. Im dargestellten Fall kann lediglich durch die Kiesfilter sowie mit den entsprechenden Filter- und den entsprechenden Vollmantelrohre (16) und (17) mittels einer motorbetriebenen Pumpe (20) Gas abgesaugt werden. Um sicherzustellen, dass auch basisnahes Deponat vom Gas durchströmt wird, muss erreicht werden, dass nur die untere Filterstrecke/Kiesbett, unter der die beiden Kiesschichten trennenden Tonsperre, abgesaugt wird. Dies kann durch eine eingezogene Auskleidung (Liner) der oberen Filterstrecke realisiert werden.
Nach einer weiteren Ausgestaltung der Erfindung kann der Liner höhenverstellbar angeordnet sein (Fig. 1). Dies ist deswegen von Vorteil, dass z.B. eine obere Filterschicht (18) abgeschlossen werden kann und die Saugung so noch weiter in die Tiefe verlegt werden kann.

Schließlich können sich die Brunnen bis unter den Grundwasserspiegel erstrecken, so dass über entsprechende zusätzliche Saugleitungen oder Druckpumpen Grundwasserproben entnommen werden können. Die beiden Brunnen (13) und (14) erstrecken sich bis unterhalb des Deponiegrundes und können unterhalb des Grundwasserspiegels enden, um über geeignete Pumpen Wasserproben entnehmen zu können.

Vorzugsweise sind über die gesamte Deponiefläche, die mehrere Hektar betragen kann, gleichmäßig Brunnen (13, 14) bzw. Saugleitungen verteilt, die mit einer einzigen oder mehreren Sauganlagen verbunden sind. Die Saugleistung der Gesamtanlage wird über frequenzumrichtergesteuerte Saugmotoren erreicht.

Durch individuelle Regulierung der Saugleistung sowie vorhandene Ventile in jeder Saugleitung kann der Gasfluss so gesteuert werden, dass sich in dem Haufwerk (11) ein etwa gleichmäßiger Unterdruck einstellt. Dies wird erreicht, indem die Saugleistung so hoch gewählt wird, dass mehr Gas abgefordert wird als durch Zersetzungsprozesse entsteht. Bisher wurden Saugleistungen erzielt, die ca. um den Faktor 30 höher lagen, als die vor Beginn der Aerobisierung gemessenen Gasbildungsraten unter anaeroben Bedingungen. Infolge der Luftdurchlässigkeit der Abdeckung (12) strömt von der Außenumgebung der Deponie Luft und damit als Bestandteil hiervon Sauerstoff in das Haufwerk (11) ein. Diese Luft muss zwangsweise das Deponiegut durchlaufen, so dass - abgesehen von geringvolumigen Probeentnahmen - die Gasabsaugung auf den über dem Deponiegrund (21) liegenden Bereich, beispielsweise das untere Drittel der Schichthöhe lokal begrenzt wird. Über die Durchflussmengenregulierung in jeder Saugleitung kann in weiten Teilen des Deponiekörpers ein gesteuerter Gasaustausch erzielt werden.

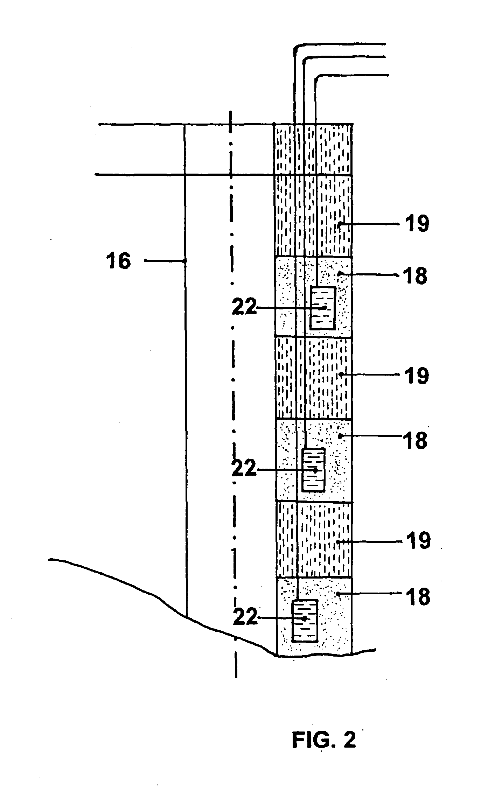

Die über die Filterkerzen (22) vorzugsweise in jedem der vorhandenen Brunnen entnommenen Gasproben bzw. oben im Brunnenkopf aus dem abgesaugten Gas erfassten Gaskonzentrationen oder vorzugsweise aus separat errichteten Multilevelbrunnen (Fig. 2) lässt sich die Gaszusammensetzung und die pro Zeiteinheit entstehende Deponiegasmenge in räumlicher Hinsicht bestimmen, so dass bei etwaigen Abweichungen vom gewünschten optimalen Zustand lokal die abgesaugte Deponiegasmenge gesteigert oder verringert werden kann. Die Messfühler (22) bzw. Filterkerzen liefern somit die jeweilige Stellgröße für die aufzuwendende Pumpleistung.

Um ggf. den wirksamen Absaugbereich weiter nach unten verschieben zu können, kann ggf. ein in der Höhe verstellbares Vollmantelrohr in einem Absaugbrunnen verschiebbar angeordnet sein, mit dem eine Gasansaugung in dem Bereich der oberen Kiesschüttung (18) ausgeschlossen werden kann und die Saugung auf die untere Kiesschüttung (18) begrenzt wird. Ob ein solches verschiebbares Rohr vorgesehen ist bzw. in welcher Höhe über dem Deponiegrund (21) die im wesentlichen einzig relevante Gasabsaugung vorgenommen wird, richtet sich im wesentlichen nach der Gasdurchlässigkeit des Haufwerks (11) der maximal möglichen Absaugleistung sowie der Art der Zersetzung, die noch anaerob und/oder aerob ablaufen kann. Zur Beschleunigung des Abbaus der Organik im Haufwerk (11) wird jedenfalls eine möglichst einheitliche aerobe Zersetzung angestrebt, bei der der Abbau um ein Vielfaches schneller als bei einer anaeroben Zersetzung abläuft.

Nach einer weiteren Ausgestaltung der Erfindung wird die Gaszusammensetzung sowie der sich jeweils in unterschiedlichen Tiefen einstellende Unterdruck im Deponiekörper über separate Multilevelbrunnen erfasst (Fig.2). In eine nicht verrohrte Bohrung werden schichtweise, vorzugsweise jeweils in 1-Meterschichten Kies(18) und bindiger Ton (19) eingebracht. In den Kiesschichten wird während der Verkiesung jeweils eine Filterkerze mit einem Messgasschlauch bis zur Geländeoberkante eingebaut. Aufgrund der Tonschichten sind die einzelnen Kiesschichten pneumatisch voneinander getrennt.

Über die einzelnen Messgasschläuche kann Gas aus den einzelnen, diskreten Schichten zur Analyse abgezogen werden. Zusätzlich kann der Unterdruck in den einzelnen Schichten erfasst werden.

In einem konkreten Ausführungsbeispiel betrug die Gasbildungsrate unter anaeroben Bedingungen ca. 30 m3/h. Die Methankonzentration betrug ca. 30 Vol%. Das Gas wurde aus einer Absauganlage mit 28 Gasbrunnen gewonnen.

Über 2 neu errichtete Gasbrunnen mit basisnaher Absaugung wurden anfänglich 100 m3/h gefördert, danach wurde die Saugleistung auf ca. 500 m³/h angehoben. Die anfängliche CH4-Konzentration betrug zunächst ca. 50 Vol%. Durch Steigerung der Absaugleistung auf 400 bis 500 m3/h fiel die Methankonzentration im Deponiegas stetig auf unter 5 Vol%. In derselben Zeit fiel der CO2-Gehal im abgesaugten Deponiegas von ca. 25 Vol% unbedeutend nur auf 22 Vol% ab. Sauerstoff war nicht nachweisbar. Der gesamte Sauerstoff wurde aerob zu CO2 umgesetzt. Die Verringerung des Methangehaltes beruht zum einen auf der Überführung mit Luftsauerstoff durchströmter Bereiche vom anaeroben in den aeroben Zustand und somit der Verringerung der Methanbildung, zum anderen kommt auch ein Verdünnungseffekt zum Tragen. Die CO2-Konzentration beruht zum grössten Teil auf der Umsetzung biogener Organik mit dem eingetragenen Luftsauerstoff.

Ziel dieser Aerobisierungsmaßnahme ist die Überführung der anaeroben, unter Methanbildung einhergehenden Umsetzung der Organik in eine viel schnellere und intensivere aerobe, unter Kohlendioxid einhergehende Umsetzung.

Kritische Emissionspfade werden dadurch unterbunden, dass einerseits Explosionsgefahren durch Methan verhindert werden und andererseits durch einen raschen aeroben Abbau die im Sickerwasser gelösten Kohlenstoffe, gemessen als CSB, stark reduziert werden.

Die beschriebenen Maßnahmen sind sowohl für Grubendeponien als auch für Haldendeponien geeignet.

Die vorliegende Erfindung umfaßt auch solche Anwendungsbereiche, in denen, insbesondere bei Deponien mit großer (Haufwerk-)Höhe, das Deponat durch Zwischenlagen aus bindigem Material (Lehm, Ton oder ähnliche Stoffe) in mehrere "Horizontalbereiche aufgeteilt ist". In solchen Fällen muß jeder Horizontalbereich als "getrennte Deponie" angesehen werden. Durch Vorversuche, in denen eine Gasabsaugung durchgeführt und spezifische Gaskonzentrationsmessungen durchgeführt werden, werden dann die einzelnen Horizontalbereiche ermittelt, in denen dann entsprechend der erfindungsgemäßen Verfahrensführung Gasabsaugungen durchgeführt werden.

## Patentansprüche

1. Verfahren zur Beschleunigung des Abbaus biogener Organik in Abfalldeponien, in denen die Abfälle in einem auf einem Deponiegrund (21) aufgeschichteten Haufwerk (11) gelagert sind, in das mindestens eine Saugleitung eingezogen ist, über die in dem Haufwerk entstehenden Deponiegase abgesaugt werden,
**dadurch gekennzeichnet,**
**dass** die im Bereich des Deponiegrundes abgesaugte Gasmenge größer ist als die in diesem Bereich durch Zersetzung entstehende Gasmenge, so dass über die Außenluft Sauerstoff in das Haufwerk (11) eindringt und der Zersetzungsprozess in eine zumindest weitgehend aerobe Zersetzung überführt wird.

2. Verfahren nach Anspruch 1, **dadurch gekennzeichnet, dass** die Gasabsaugung ausschließlich im Bereich des Deponiegrundes, vorzugsweise im unteren Drittel des Haufwerkes (11) und/oder in einer sich bis zu 6 m über den Deponiegrund (21) erstreckenden Zone durchgeführt wird.

3. Verfahren nach Anspruch 1 oder 2, **dadurch gekennzeichnet, dass** die Gasabsaugung an mehreren, im Deponiegrundbereich angeordneten Orten, die vorzugsweise äquidistant zueinander liegen, durchgeführt wird.

4. Verfahren nach einem der Ansprüche 1 bis 3, **dadurch gekennzeichnet, dass** die Saugleistung in Abhängigkeit der in unterschiedlichen Höhen im Haufwerk gemessenen Gaszusammensetzung so gesteuert wird, dass an jedem Ort im Haufwerk ein für eine aerobe Zersetzung notwendiger Sauerstoffgehalt eingestellt ist.

5. Vorrichtung zur Durchführung des Verfahrens nach einem der Ansprüche 1 bis 4, mit mindestens einer in das Haufwerk eingezogenen, sich bis zum Deponiegrundbereich erstreckenden Saugleitung, mindestens einem Messfühler zur Analyse und Messung der Konzentration der im Haufwerk entstehenden Gase und einer Steuervorrichtung zur Einstellung der Pumpleistung, nämlich der Durchflussmenge der abgesaugten Gase in der oder den Saugleitungen in Abhängigkeit der über den oder die Messfühler festgestellten Gase und deren Konzentration, **dadurch gekennzeichnet, dass** die Absaugöffnung der Saugleitung(en) im Bereich des Deponiegrundes angeordnet ist/sind, nämlich im unteren Drittel des Haufwerkes.

6. Vorrichtung nach Anspruch 5, **dadurch gekennzeichnet, dass** in dem Haufwerk (11) mehrere, vorzugsweise zueinander äquidistant angeordnete Saugleitungen eingezogen sind.

7. Vorrichtung nach Anspruch 5 oder 6, **dadurch gekennzeichnet, dass** mehrere Saugleitungen installiert sind, deren Lage, Abstand und deren Pumpleistung so gewählt ist, dass sich deren Absaug-Wirkbereiche zumindest berühren, vorzugsweise geringfügig überlappen.

8. Vorrichtung nach Anspruch 7, **dadurch gekennzeichnet, dass** die Pumpleistung in jeder Saugleitung individuell regelbar ist, vorzugsweise in Abhängigkeit der im betreffenden Wirkbereich gemessenen Gaskonzentration steuerbar ist.

9. Vorrichtung nach einem der Ansprüche 5 bis 8, **dadurch gekennzeichnet, dass** die Saugleitungen (Filterstrecken) in ihrem oberen Bereich einen gasdichten Mantel, ggf. über eine eingezogene Auskleidung (Liner) besitzen und nur in ihrem unteren Bereich ein oder mehrere Gasansaugöffnung(en) besitzen.

10. Vorrichtung nach einem der Ansprüche 5 bis 9, **dadurch gekennzeichnet, dass** sich die Saugpegel bis unter den Grundwasserspiegel erstrecken.

11. Vorrichtung nach Anspruch 5, **dadurch gekennzeichnet, dass** zur Messung der Gaszusammensetzung in unterschiedlichen Tiefen, Filterkerzen zur Gasprobennahme in einer Bohrung in vorzugsweise 1 m mächtigen Kiesschichten eingebettet sind, bei der vorzugsweise jeweils 1 m dicke Lagen aus Filterkies und Ton übereinandergeschichtet sind.

## Claims

1. Method for accelerating the decomposition of biogenic organic matter in waste disposal sites, in which the waste is deposited upon a deposit ground in a debris heap (11) in which at least one suction line is inserted via which the waste gases produced in the debris heap are sucked out,
**characterised in that**,
the quantity of gas sucked from the region of the bottom of the deposit ground is greater than the gas quantity arising in this region from decomposition, so that oxygen from the outside air penetrates into the debris heap (11) and transforms the decomposition process into an, at least to a large extend, aerobic decomposition.

2. Method according to claim 1, **characterised in that** the gas suction is conducted exclusively in the region of the deposit ground, preferably in the lower third of the debris heap (11) and/or in a zone which extends to 6 meters above the deposit ground (21).

3. Method according to claim 1 or 2, **characterised in that** the gas suction is carried out at a multiplicity of locations arranged in the region of the deposit ground which lie preferably equidistant from one another.

4. Method according to one of claim 1 to 3, **characterised in that** the suction power is so controlled in dependence upon the gas composition measured at different heights in the debris heap that at each location in the debris heap an oxygen content is established as is required for an aerobic decomposition.

5. Device for carrying out the method according to one of claim 1 to 4, with at least one suction line inserted in the debris heap and extending to the deposit ground region, at least one measurement sensor for analyzing and measuring the concentration of the gases arising in the debris heap and a control device for adjusting the pump power, namely the throughflow quantity of the gas drawn off in the suction line or the suction lines as a function of the gases detected by the measurement sensor and their concentration **characterised in that** the suction opening of the suction line(s) is/are arranged in the region of the deposit ground, namely in the lower third of the dump pile or heap.

6. Device according to claim 5, **characterised in that** a plurality preferably mutually equidistantly arranged suction lines are inserted in the debris heap (11).

7. Device according to claim 5 or 6, **characterised in that** a plurality of suction lines are installed whose positions, spacing and pump capacity are so selected that their respective suction effective regions at least are adjustment and preferably overlap.

8. Device according to claim 7, **characterised in that** the pump power in each suction line is individually controllable, preferably in dependence upon the gas concentrations measured in the respective effective region.

9. Device according to one of claims 5 to 8, **characterised in that** the suction power (filter stretches) in their upper regions are limited by a gas-tight shell, optionally by a liner incorporated therein and only in their lower regions have one or more gas suction opening(s).

10. Device according to one of the claims 5 to 9, **characterised in that** the suction level extends to below the ground water level.

11. Device according to claim 5, **characterised in that** for measuring the gas position at different depths, filter cartridges for gas sample-taking are embedded in a bore in preferably one meter great gravel layers in which preferably each one meter thick layer of filter gravel and clay are disposed in layers alternately with one another.

## Revendications

1. Procédé pour accélérer la décomposition des matières organiques biogènes dans des décharges publiques de déchets dans lesquelles les déchets sont stockés dans un tas (11) lequel est empilé sur un fond (21) de la décharge et dans lequel est insérée au moins une conduite d'aspiration par l'intermédiaire de laquelle les gaz de décharge produits dans ledit tas sont aspirés, **caractérisé par le fait que** la quantité de gaz aspirée au niveau du fond de la décharge est plus importante que la quantité de gaz produite dans cette zone par décomposition, de sorte que de l'oxygène provenant de l'air extérieur pénètre dans ledit tas (11) et que le processus de décomposition est transformé en une décomposition au moins dans une large mesure aérobie.

2. Procédé selon la revendication 1, **caractérisé par le fait que** l'aspiration des gaz est réalisée exclusivement au niveau du fond de la décharge, de préférence dans le tiers inférieur du tas (11) et/ou dans une zone s'étendant jusqu'à 6 mètres sur le fond (21) de la décharge.

3. Procédé selon la revendication 1 ou 2, **caractérisé par le fait que** l'aspiration des gaz est réalisée à plusieurs endroits situés au niveau du fond de la décharge, qui, de préférence, se trouvent à distance égale les uns des autres.

4. Procédé selon l'une quelconque des revendications 1 à 3, **caractérisé par le fait que** la puissance d'aspiration est commandée en fonction de la composition de gaz mesurée à différentes hauteurs dans le tas, de telle sorte que, à chaque endroit dans le tas, est réglée une teneur en oxygène nécessaire à une décomposition aérobie.

5. Dispositif de mise en ouvre du procédé selon l'une quelconque des revendications 1 à 4, comprenant au moins une conduite d'aspiration insérée dans le tas et s'étendant jusqu'au niveau du fond de la décharge, au moins un capteur pour analyser et mesurer la concentration des gaz produits dans le tas, ainsi qu'un dispositif de commande destiné à régler la puissance de pompage, à savoir le débit des gaz aspirés, dans la ou les conduite(s) d'aspiration en fonction des gaz détectés par le ou les capteur(s) et de leur concentration, **caractérisé par le fait que** l'ouverture d'aspiration de la (des) conduite(s) d'aspiration est/sont disposée(s) au niveau du fond de la décharge, à savoir dans le tiers inférieur du tas.

6. Dispositif selon la revendication 5, **caractérisé par le fait que** plusieurs conduites d'aspiration, de préférence disposées à distance égale les unes des autres, sont insérées dans ledit tas (11).

7. Dispositif selon la revendication 5 ou 6, **caractérisé par le fait que** plusieurs conduites d'aspiration sont installées dont la position, la distance ainsi que la puissance de pompage sont choisies de manière à ce que leurs zones actives d'aspiration soient au moins en contact entre elles, de préférence se recouvrent légèrement.

8. Dispositif selon la revendication 7, **caractérisé par le fait que** la puissance de pompage dans chacune des conduites d'aspiration peut être réglée individuellement, de préférence être commandée en fonction de la concentration de gaz mesurée dans la zone active en question.

9. Dispositif selon l'une quelconque des revendications 5 à 8, **caractérisé par le fait que** les conduites d'aspiration (trajets de filtrage) possèdent, dans leur zone supérieure, une enveloppe étanche aux gaz, le cas échéant sous forme d'un revêtement inséré (gaine) et ne possèdent que dans leur zone inférieure une ou une pluralité d'ouverture(s) d'aspiration de gaz.

10. Dispositif selon l'une quelconque des revendications 5 à 9, **caractérisé par le fait que** les niveaux d'aspiration s'étendent jusqu'au-dessous du niveau de la nappe phréatique.

11. Dispositif selon la revendication 5, **caractérisé par le fait que**, pour mesurer la composition de gaz à différentes profondeurs, des bougies filtrantes pour l'échantillonnage de gaz sont enrobées dans un trou de forage dans des couches de gravier ayant de préférence une épaisseur de 1 m, dans lequel, de préférence, des couches de gravier de filtration et d'argile respectivement d'une épaisseur de 1 m sont empilées les unes sur les autres.
